# EUROPEAN PATENT SPECIFICATION

(11) **EP 3 242 865 B1**
(45) Date of publication and mention of the grant of the patent: **13.01.2021**
(21) Application number: 15830900.5
(22) Date of filing: 10.12.2015
(51) Int. Cl.: C07C 67/03, C07C 69/28

(54) **METHOD OF PREPARING ESTERS OF LACTIC AND LACTYLATIC ACID IN ALCOHOLYSIS REACTION OF ALIPHATIC POLYESTER**
VERFAHREN ZUR HERSTELLUNG VON ESTERN VON MILCH- UND LAKTYLATISCHER SÄURE IN ALKOHOLYSEREAKTION AUS ALIPHATISCHEM POLYESTER
PROCÉDÉ DE PRÉPARATION D'ESTERS D'ACIDE LACTIQUE ET LACTYLATIQUE DANS UNE RÉACTION D'ALCOOLYSE DE POLYESTER ALIPHATIQUE

(30) Priority: 08.01.2015 PL 41089815
(43) Date of publication of application: 15.11.2017
(73) Proprietor: Siec Badawcza Lukasiewicz - PORT Polski Osrodek Rozwoju Technologii, 54-066 Wroclaw (PL)
(72) Inventor: SOBOTA, Piotr, PL-54-029 Wroclaw (PL); PETRUS, Rafal, PL-57-500 Bystrzyca Klodzka (PL); BYKOWSKI, Dominik, Jerzy, PL-52-428 Wroclaw (PL)
(74) Representative: Godlewski, Piotr
(86) International application number: PCT/PL2015/000201
(87) International publication number: WO 2016/111635

(56) References cited:
- US-A- 5 264 617
- US-A1- 2003 008 927

## Description

The object of the invention is a method of preparing esters of lactic and lactylatic acid in alcoholysis reaction of aliphatic polyester. In particular, the invention relates to a method of preparing ethyl lactate with simultaneous preparation of ethyl lactyllactate.

Alkyl esters of lactic acid are a group of chemical compounds used in a number of industrial sectors due to their unique physicochemical properties: high boiling temperatures (144 - 246°C), low vapour pressures (0.002 - 0.34 kPa, 20 °C), high miscibility with other solvents (water, alcohols, esters and oils) and low toxicity.

Alkyl esters of lactic acid belong to the group of natural environment-friendly solvents prepared from renewable sources (biomass), called "green solvents". They are an alternative for solvents from petrochemical origin. The can be easily recycled and subjected to ultimate biodegradation.

On a large-scale volume, there are only few esters of lactic acid produced, such as: methyl lactate, ethyl lactate, n-propyl lactate, isopropyl lactate, n-butyl lactate and 2-ethylhexyl lactate (companies: GALACTIC, PURAC). Those compounds are used mainly as: solvents for cellulose and its derivatives, natural resins, synthetic polymers, dyes, oils, paints, varnishes and glues or components of surfactant compositions. Ethyl lactate has special place among them.

Ethyl lactate (ElacH) is colourless, high-boiling liquid (154°C) of characteristic, butter-fruity aroma. In small amounts it occurs naturally in food products, in particular in chicken, wine and fruits. It has two enantiomers: L-ElacH and D-ElacH. It has a wide range of applications in the industry, where it is use inter alia in manufacturing of: cleaners and safeners, scented products, industrial lubricants, liquids for metal working, antifreezes and de-icing agents, heat transmission liquids, paint, varnish, graffiti and glue removers or bio-solvents. High-purity ethyl lactate is use, inter alia, in electronics sector as a solvent, cleaner and de-greasing agent for electronic assemblies, and in medical sector e.g. for stent manufacturing. It does not affect the change of biological activity of pharmacologically active substances, therefore it is successfully applied as a solvent or dispersant in pharmaceutical compositions. In industrial chemistry, it can be use as a replacer for toluene, xylene, acetone or N-methylpyrrolidone.

The method of preparing esters of lactic acid at the industrial scale, and particularly ethyl lactate, comprising esterification of lactic acid with alcohol in the presence of acid catalysts, is known in the art (US2434300, US7297809, US20060041165, CN1102180, US20110160480, US2610206). Excess alcohol is used in order to increase the yield of the esterification. The main disadvantage of process applied is the separation of products that is technologically complicated due to the presence of water and alcohol in reaction mixture (US2465772). For example, ternary azeotropic mixture H₂O/EtOH/EtLac is formed in a process of ethyl lactate synthesis and its individual components are separated as a result of filtering through a hydrophobic membrane or reactive distillation.

Another possibility includes dehydration of reaction mixture with absolute ethanol. In this method, water is removed by distilling off H₂O/EtOH/EtLac (CN1102180) azeotrope. Difficulties associated with isolation of ethyl lactate are limited by using as a substrate in esterification process low molecular weight oligomers of polylactide (PLA) - biodegradable polymer from bio-mass, obtained as a result of polycondensation of lactic acid or a mixture of oligomers and lactic acid of various composition (US20030008927). Yet another solution involves synthesis of alkyl lactates in the process of esterification of high purity lactic acid obtained by hydrolysis of polylactide (WO02060852).

Esterification reaction can be also carried out using ammonium, sodium, potassium, calcium lactates (US2406648, US5071754; US5210296; US5723639; EP614983; Ind. Eng. Chem., 1952, 44(9), 2189-2191; J. Am. Chem. Soc., 1953, 75(5), 1242-1244; US8674131; US2406648, US8674131) or products of lactic fermentation of carbohydrates (US20130274505, US20140024853, EP0614983, US20130231497;Chem. Rev. 2014, 114, 1909-1971) as a source of lactic acid.

Lactic acid esters can be also obtained using less popular methods of synthesis e.g. in reactions of: alcoholysis of lactides (US2410740, Tetrahedron Lett., 2006, 47, 6517-6520; Dalton Trans., 2008, 3048-3050; 2011, 40, 4042-4044; US20090200511; WO2012036693; US2371281, US20090173917), glycerol with alcohols (US20130079547), chloroalkanes or chloroalkenes with sodium lactate (US2315168, US2367798), acetaldehyde cyanohydrin with water and alcohols (US1650950), lactic acid amids with alcohols (US5824818).

One of the least studied methods of synthesis of lactic acid esters is polylactide alcoholysis. Above method implies that in the presence of alcohol polymer is degraded to oligo- and monoesters of lactic acid. This solution enables for chemical recycling of aliphatic polyesters and polyhydroxy acids and it solves the problem of raw materials because of usage of packagings and food foils as a substrate. The main assumptions of the method may be used also for production of high purity lactic acid or lactide as a result of PLA depolymerisation in the presence of water (US20120142958, US20120165554).

From the publication by Fliedel C. et al. (Chem. Cat. Chem. 2014, 6, 1357-1367) the method is known for producing methyl lactate, comprising polymerisation of rac-LA in the presence of alkoxy zinc complexes [(S,C_{NHC})ZnCl(OBn)]₂ and [(O,C_{NHC})ZnCl(OBn)]₂ stabilised with N-heterocyclic carbenes, which leads to the formation of atactic PDLLA that is used as a substrate to produce methyl lactate. In the reaction of PDLLA (8 kDa) with MeOH in the presence of [(S,CNHC)ZnCl(OBn)]₂ at the stoichiometry of reagents lac/Zn/MeOH = 100/1/1000, polymer conversion to rac-methyl lactate was 90% in 1h, under atmospheric pressure in CH₂Cl₂ solvent. However, by using PLLA (18 kDa) as a commercial substrate, 25% conversion of polymer to methyl L-lactate was obtained at stoichiometry of reagents lac/Zn/MeOH = 200/1/100. The disadvantages of this method are long reaction times when commercial polymer (PLLA, 24 h) is used as a substrate, use of CH₂Cl₂ as a solvent, use of catalyst prepared in three-stage synthesis from very expensive substrates, no elaborated methods for catalyst neutralisation, isolation and purification of final product.

From publication by Whitelaw E.L. et al. (Chem. Commun., 2011, 47, 10004-10006) a method is known, where titanium, zirconium and hafnium complex compounds are used as initiators for rac-LA polymerisation process and catalysts of polylactide alcoholysis. In the reaction of PLLA (211 kDa) with MeOH with stoichiometry of reagents lac/Hf/MeOH = 100/1/500, at room temperature, under atmospheric pressure, in CH₂Cl₂ solvent, during 24h the 74% conversion of PLLA to methyl L-lactate was obtained in the presence of [Hf(L₁)(OⁱPr)₂], where L₁ = (2-O-C₆H₃Me-3)CH=NCH₂CH₂N(Me)CH₂(2-O-C₆H₂-But₂- 3,5),. However under similar conditions but with reagents stoichiometry lac/Hf/MeOH = 100/1/100 the PDLLA (10 - 45 kDa) conversion to methyl rac-lactate achieved 11-18%. The disadvantages of this method are long reaction times (24 h), use of CH₂Cl₂ as a solvent, use of catalyst prepared in complex synthesis from expensive substrates, no elaborated methods for catalyst neutralisation, isolation and purification of final product.

From publication by Carné Sanchez A. et al. (Eur. Polym. J., 2011, 47, 1970-1976) synthesis is known, where 70% conversion of PLLA to methyl lactate was obtained in the presence of [Zn(CH₃COO)₂]·2H₂O, with reagents stoichiometry lac/Zn/MeOH = 70/1/371, at 64.7 °C under atmospheric pressure during 15h. Also, 21% conversion of PLLA (200kDa) to ethyl lactate was obtained in the presence of [Zn(CH₃COO)₂]·2H₂O, with reagents stoichiometry lac/Zn/EtOH = 70/1/257 under the same reaction conditions.

The disadvantages of this method are long reaction times (15 h), use of catalyst in a form of hydrate, which causes lactic acid formation, carrying out reaction in solvents with low boiling temperatures (MeOH, EtOH), no elaborated methods for catalyst neutralisation, isolation and purification of final product.

From publication US5264617 known is the reaction where 87% conversion of PLLA (200 kDa) to ethyl L-lactate was obtained in the presence of H₂SO₄ with reagents stoichiometry lac/H₂SO₄/EtOH = 267/1/513, at 150 °C under autogenous pressure of reagents, through 2h, using pressure reactor.

From publication US2012029228 process is known where 61% conversion of PLLA to ethyl L-lactate was obtained in the presence of TBD, with reagents stoichiometry of lac/TBD/EtOH = 200/1/400, at 138 °C in pressure reactor under a pressure of 2.6 - 2.8 bar, during 20h.

Publication by Filachione E.M. (Ind. Eng. Chem., 1945, 37, 388-390) discloses the method where 57% conversion of PLLA oligomers to ethyl L-lactate was obtained in the presence of H₂SO₄, with reagents stoichiometry lac/H₂SO₄/EtOH = 40/1/100, at 100 °C, in sealed reaction vessel (bottle), during 4h. On the other hand, 77 % conversion of PLLA oligomers to ethyl lactate was obtained by bubbling EtOH vapours through column filled with PLA in the presence of H₂SO₄ during 2.9 hours, at the temperature range of 120-126 °C.

The aim of the invention is to provide improved process for preparing esters of lactic acid, particularly ethyl lactate, where the conversion of aliphatic polyester to lactic acid esters is 100%, while the yield of synthesis of lactic acid ester is higher than 95%, preferably 99%. The aim of the invention is also to provide the technology for preparing lactic acid esters with simultaneous production of desired product being esters of lactylatic acid, particularly ethyl lactyllactate. At the same time, the aim of the invention is to provide a method for production of lactic acid esters, that will enable to carry out the reaction without substrate loss, with high yield, in a short time and in substantially simplified way without laborious stages of purifying the mixture of reagents from unwanted components, i.e. catalyst, solvent or unreacted substrate. The aim of the invention is therefore to provide a method for preparation of above products, characterised in 100% substrates conversion and high products yield, without catalyst.

The aim of undertaken research was also to elaborate technology of chemical conversion of PLA to high purity ethyl lactate and ethyl lactyllactate in solvothermal conditions.

Unexpectedly, above aims are realised in the present invention.

The object of the invention is a method for preparing esters of lactic and lactylatic acid, where the conversion of substrates is 100%, comprising:
a) placement in pressure reactor polylactide (PLA) and alcohol (ROH) wherein the alcohol is selected from methyl, ethyl, n-propyl, isopropyl, n-butyl, isobutyl, sec-butyl, tert-butyl, n-pentyl, isopentyl, n-hexyl, 2-ethylhexyl, n-octyl, allyl, propargyl, benzyl alcohol or mixture of alcohols
   with ratio of reagents from 2 to 10 molar equivalents of alcohol for structural unit of polymer ROH/mer;
b) optionally, introducing pre-catalyst to reaction mixture *in situ* wherein the catalyst is prepared in direct one-stage synthesis from organometallic compounds, metal halides, amides, hydrides, carboxylates, acetylacetonates, alcoholates, aryloxanes, metal oxides, metallic dusts, metallic chips, low melt metal alloys and catalytic compositions composed of mixtures of said precursors in the reaction with ROH in solvothermal conditions during the process;
   and wherein the catalyst is synthetized from hydroxides, organometallic compounds, metal alkoxides, salts of inorganic acids
c) carrying out the alcoholysis reaction of polylactide in the presence of alcohol or mixture of alcohols, under autogenous pressure of reaction mixture components within a range from atmospheric pressure to 107 bar, in suitably selected temperature, wherein
   i. in non-catalyst reaction, the reaction temperature is 220-260°C,
   ii. while in catalyst reaction, the reaction temperature is 150-220°C,
d) separating and purifying final product by filtration, evaporation and distillation.

Preferably, in non-catalyst reaction, the reaction temperature is 240-260°C, and in catalyst reaction, the reaction temperature is 180-220°C.

Preferably, polylactide (PLA) is selected from the group comprising high molecular weight polylactide, such as isotactic poly(L-lactide) or poly(D-lactide) and atactic poly(D,L-lactide), as well as low molecular weight polylactide.

Preferably, ester of lactic acid is ethyl lactate and ester of lactylactic acid is ethyl lactylactate. Preferably, polylactide is introduced to the reactor in a form of granules or wastes of postconsumer PLA.

Preferably, alcoholysis of polylactide is conducted under inert atmosphere in solvothermal conditions.

Preferably, alcoholysis of polylactide is conducted in N₂ or noble gases.

Preferably, the reaction is conducted in air.

Preferably, the reaction time is 1 h.

Preferably, metallic or organometallic pre-catalyst is used in the reaction selected from the group comprising metals of main groups, transition elements and lanthanides, as well as alloys of two and more metals in a composition composed of a mixture of said elements or their organometallic compounds, halides, amides, hydrides, carboxylates, acetylacetonates, metal oxides and their mixture with metallic dust, metallic chips.

Preferably, organometallic pre-catalyst in a form of solution or in fine crystalline form is used in the reaction.

Preferably, catalyst is used in the amount of 0.1 to 5% mol per polymer structural unit.

The solution according to the invention provides new, simple and environmentally friendly method for chemical recycling of aliphatic polyesters, especially polylactide, to lactic acid esters with formation of lactylatic acid esters. The process is conducted in solvothermal conditions, it does not require additional solvents and therefore it does not cause pollution of the environment and atmosphere. The solution according to the invention enables to conduct a reaction without catalyst, while maintaining high conversion of substrates to products, which enables to omit the stages of catalyst neutralisation and removal, and in the opposite to methods where organic or acid catalysts are used, it does not require either purification of reaction mixture from water and unreacted lactic acid. No raw materials are lost during the process (atomic yield 100%).

The process can also operates in the presence of catalyst. Substrates, products and catalyst recovered from reaction mixture at purification stage can be reused in technological process. Liquid components of the reaction mixture can be distilled-off directly from the reactor - firstly alcohol (which can be reused in synthesis), next lactic acid esters and finally lactylactic acid esters, and catalyst that forms solid phase can be reused in next catalytic cycle. Developed invention can be used for continuous synthesis of esters of lactic and lactylatic acid provided that reagents are introduced to the reactor continuously and mixture of ester and alcohol is removed continuously.

As a substrate, mixture of PLA and other polymers, such as: PET, PP, PE, PVC and PC, which are non-reactive and insoluble in the conditions of process of the invention, can be used for reaction according to the invention.

It is preferred to use anhydrous alcohols, but it is acceptable to use also alcohols containing water or mixture of alcohols with other solvents.

The solution according to the invention enables also to conduct reaction for preparing esters of lactic acid in the presence of catalyst.

Particularly preferred is preparing used catalysts *in situ* in direct one-stage synthesis from organometallic compounds; metal halides, amides, hydrides, carboxylates, acetylacetonates, alcoholates, aryloxanes, metal oxides; metallic dusts; metallic chips; low melt metal alloys and catalytic compositions composed of mixtures of said precursors in the reaction with ROH in solvothermal conditions during the process.

Main group and transition metal compounds of [M(OR)ₙ], [M(OR)ₙ(L)ₘ] or [M(L)ₙ(ROH)_{X}] type were used as catalysts for PLA alcoholysis process, where M = metal, -OR = alkoxide group, L = functional ligand (acac, O²⁻, RCOO⁻, Cl⁻), n, m - factors dependant from valence of the metal from the range of 1 - 4, ROH - alcohol, x - numerical factor showing the number of ROH ligands coordinated to central atom, its value depends on metal, as well as donor and steric effect of L ligands.

The following groups of compounds were used for the synthesis of catalysts:
- metals: Na, K, Mg, Sn, Zn, Ca, Sr, Ba, Li, La and others;
- alloys: Sn/Pb and others;
- organometallic compounds: MgBu₂, ZnEt₂, LiMe, SnEt₂Cl₂, TiCp₂Cl₂, ZrCp₂Cl₂, FeCp₂ and others;
- amides: K(N(SiMe₃)₂ and others;
- hydroxides KOH, NaOH and others;
- metal alkoxides: Ca(OMe)₂, Ba(OⁱPr)₂, NaOEt, KOEt, Ti(OMe)₄, Zr(OEt)₄, Al(OEt)₃, Cu(OMe)₂, VO(OEt)₃ and others;
- metal oxides: MgO, CaO, Al₂O₃ and others;
- metal acetylacetonates: Cr(acac)₃, VO(acac)₂, Mn(acac)₂ and others;
- metal halides: SnCl₂, ZnCl₂, CaCl₂ and others;
- metal carboxylates: Sn(Oct)₂, Ag(OAc) and others;
- salts of inorganic acids: NaVO₃ and others.

Preferred is the use of catalysts that are highly reactive, human and environment-friendly, safe to housing and storage, such as e.g. Mg²⁺, Ca²⁺, Zn²⁺, K⁺, Na⁺ alkoxides.

Catalysts used in the reaction of the invention are prepared *in situ,* show very high activity, due the fact they do not aggregate and do not cause side reactions of polycondensation or epimerisation of lactic acid esters.

The presence of catalyst selected in such a way in reaction mixture at the stage of final product isolation does not cause polycondensation of obtained esters.

Developed synthesis method may be used for preparing lactic acid esters of various optical activity that is determined by the type of microstructure of used polylactide. For example, using isotactic poly(L-lactide) (abbreviation: PLLA) one can prepare esters of L-lactic acid; poly(D-lactide) (PDLA) - esters of D-lactic acid. While esters of L,D-lactic acid (racemic mixture) may be prepared with the use of atactic, syndiotactic, heterotactic, isotactic stereoblock poly(L,D-lactide) (PDLLA) or equimolar mixture of PLLA and PDLA as a substrate. Catalysts used in alcoholysis reaction of PLA, especially magnesium and calcium, do not cause changes in optical activity of synthesited esters.

In order to prepare high purity-ethyl lactate and other esters of lactic acid, distillation process should be conducted under atmospheric pressure or under reduced pressure in order to remove excess of alcohol that can be used for next synthesis. Preferred is to distill off alcohol at 35- 65 °C under a pressure in a range from 2 - 50 mBar. Ester is purified by distillation under atmospheric or reduced pressure. Preferred is to distill ester under reduced pressure at 55 - 70 °C under a pressure in a range from 2 - 50 mBar.

Developed technology may be used for synthesis of alkyl, alkenyl, alkynyl, cycloalkyl, cycloalkenyl, cycloalkynyl and aryl hydroxy acids (C2-C9) esters, and particularly: glycolic, 6-hydroxycaproic, (2-hydroxyetoxy)acetic, 3-hydroxypropionic, 3-hydroxybutyric, 4-hydroxybutyric, 3-hydroxyvaleric, 5-hydroxyvaleric, 3-hydroxycaproic, 3-hydroxycaproic, 3-hydroxydecanoic acid and many other hydroxyalkanoates, wherein aliphatic polyesters are used as substrates, and in particular: polyglycolide (PGA), polycaprolactone (PCL), polydioxanone (PDO), poly(3-hydroxypropionate) (PHP), poly(3-hydroxybuturate) (PHB), poly(4-hydroxybutyrate) (PBL), poly(3-hydroxyvalerate) (PHV), poly(5-hydroxyvalerate) (PVL), poly(3-hydroxyhexanoate) (PHH), poly(3-hydroxyoctanoate) (PHO), poly(3-hydroxydecanoate) (PHD) and other polyhydroxyalkanoates (PHA) and their copolymers and mixtures.

The invention is illustrated by the following embodiments.

### Example 1 The reactions for preparing ethyl lactate and ethyl lactyllactate without catalyst

### 1.1

10.0 g of poly(L-lactide) (100 eq.) in a form of granules from NatureWorks Ingeo™ Biopolymer 2003D and 88.5 ml of anhydrous ethyl alcohol (1000 eq.) from Chempur were placed in 150 ml pressure reactor under N₂. The reactions were carried out at the temperature of 240 - 260 °C for 1h under autogenous pressure of reagents (in a range from atmospheric pressure to 107 bar) with a ratio of [lac_{(PLLA)}]/[EtOH] = 1/10. Conversion of PLLA to lactic acid esters was 100%. The yield of synthesis was 99% for ethyl lactate and 1% for ethyl lactyllactate.

### 1.2

10.0 g of poly(poly(L-lactide) (100 eq.) in a form of granules from NatureWorks Ingeo™ Biopolymer 2003D and 32.5 ml of anhydrous ethyl alcohol (400 eq.) from Chempur were placed in 150 ml pressure reactor under N₂. The reactions were carried out at the temperature of 240 - 260 °C for 1h under autogenous pressure of reagents (in a range from atmospheric pressure to 42 bar) with a ratio of [lac_{(PLLA)}]/[EtOH] = 1/4. Conversion of PLLA to lactic acid esters was 100%. The yield of synthesis was 96% for ethyl lactate and 4% for ethyl lactyllactate. Optical rotation for ethyl L-lactate is -14.4.

### 1.3

10.0 g of poly(L-lactide) (100 eq.) in a form of granules from NatureWorks Ingeo™ Biopolymer 2003D and 16.5 ml of anhydrous ethyl alcohol (200 eq.) from Chempur were placed in 150 ml pressure reactor under N₂. The reactions were carried out at the temperature of 240 - 260 °C for 1h under autogenous pressure of reagents (in a range from atmospheric pressure to 28 bar) with a ratio of [lac_{(PLLA)}]/[EtOH] = 1/2. Conversion of PLLA to lactic acid esters was 100%. The yield of synthesis was 90 % for ethyl lactate and 10% for ethyl lactyllactate.

### 1.4

5.0 g of atactic poly(D,L-lactide) (100 eq.) in a form of granules from Biomatpol (140 kDa) and 16.5 ml of anhydrous ethyl alcohol (400 eq.) from Chempur were placed in 150 ml pressure reactor under N₂. The reactions were carried out at the temperature of 220 °C for 1h under autogenous pressure of reagents (in a range from atmospheric pressure to 28 bar) with a ratio of [lac_{(PDLLA)}]/[EtOH] = 1/4. Conversion of PDLLA to lactic acid esters was 100%. The yield of synthesis was 98 % for ethyl lactate and 2% for ethyl lactyllactate. Optical rotation for ethyl rac-lactate is -0.2.

### 1.5

10.0 g of poly(D-lactide) (100 eq.) in a form of granules from Purac PURASORB® PD24 and 32.5 ml of anhydrous ethyl alcohol (400 eq.) from Chempur were placed in 150 ml pressure reactor under N₂. The reactions were carried out at the temperature of 220 °C for 2h under autogenous pressure of reagents (in a range from atmospheric pressure to 28 bar) with a ratio of [lac_{(PDLA)}]/[EtOH] = 1/4. Conversion of PDLA to lactic acid esters was 100%. The yield of synthesis was 97 % for ethyl lactate and 3% for ethyl lactyllactate. Optical rotation for ethyl D-lactate is +14.0.

### Example 2 The reactions for preparing ethyl lactate and ethyl lactyllactate in the presence of catalyst

### 2.1

10.0 g of poly(L-lactide) (100 eq.) in a form of granules from NatureWorks Ingeo™ Biopolymer 2003D, 88.5 ml of anhydrous ethyl alcohol (1000 eq.) from Chempur and 1.39 ml 1.0 M solution of MgnBu₂ in hexane from Sigma-Aldrich (1 eq.) were placed in 150 ml pressure reactor under N₂. The reactions were carried out at the temperature from 180 to 220 °C for 1h under autogenous pressure of reagents (in a range from atmospheric pressure to 40 bar) with a ratio of [lac_{(PLLA)}]/[Mg]/[EtOH] = 100/1/1000. Conversion of PLLA to lactic acid esters was 100%. The yield of synthesis was 99 % for ethyl lactate and 1% for ethyl lactyllactate.

### 2.2

10.0 g of poly(L-lactide) (100 eq.) in a form of granules from NatureWorks Ingeo™ Biopolymer 2003D, 88.5 ml of anhydrous ethyl alcohol (1000 eq.) from Chempur and 1.39 ml 1.0 M solution of MgnBu₂ in hexane from Sigma-Aldrich (1 eq.) were placed in 150 ml pressure reactor under N₂. The reactions were carried out at the temperature of 160 °C for 1h under autogenous pressure of reagents (in a range from atmospheric pressure to 14 bar) with a ratio of [lac_{(PLLA)}]/[Mg]/[EtOH] = 100/1/1000. Conversion of PLLA to lactic acid esters was 100%. The yield of synthesis was 91 % for ethyl lactate and 9% for ethyl lactyllactate.

### 2.3

10.0 g of poly(L-lactide) (100 eq.) in a form of granules from NatureWorks Ingeo™ Biopolymer 2003D, 32.5 ml of anhydrous ethyl alcohol (400 eq.) from Chempur and 1.39 ml 1.0 M solution of MgnBu₂ in hexane from Sigma-Aldrich (1 eq.) were placed in 150 ml pressure reactor under N₂. The reactions were carried out at the temperature from 150 to 220 °C for 1h under autogenous pressure of reagents (in a range from atmospheric pressure to 25 bar) with a ratio of [lac_{(PLLA)}]/[Mg]/[EtOH] = 100/1/400. Conversion of PLLA to lactic acid esters was 100%. The yield of synthesis was 99 % for ethyl lactate and 1% for ethyl lactyllactate.

### 2.4

10.0 g of poly(L-lactide) (100 eq.) in a form of granules from NatureWorks Ingeo™ Biopolymer 2003D, 16.5 ml of anhydrous ethyl alcohol (200 eq.) from Chempur and 1.39 ml 1.0 M solution of MgnBu₂ in hexane (1 eq.) from Sigma-Aldrich were placed in 150 ml pressure reactor under N₂. The reactions were carried out at the temperature from 180 to 220 °C for 1h under autogenous pressure of reagents (in a range from atmospheric pressure to 16 bar) with a ratio of [lac_{(PLLA)}]/[Mg]/[EtOH] = 100/1/200. Conversion of PLLA to lactic acid esters was 100%. The yield of synthesis was 95% for ethyl lactate and 5% for ethyl lactyllactate.

### 2.5

1.5 kg of poly(L-lactide) (100 eq.) in a form of granules from NatureWorks Ingeo™ Biopolymer 2003D, 2.4 dm³ of anhydrous ethyl alcohol (200 eq.) and 5.4 g of metallic magnesium (1 eq.) from Chempur were placed in 5 dm³ pressure reactor under N₂. The reactions were carried out at the temperature of 200 °C for 1h under autogenous pressure of reagents (in a range from atmospheric pressure to 17 bar) with a ratio of [lac_{(PLLA)}]/[Mg]/[EtOH] = 100/1/200. Conversion of PLLA to lactic acid esters was 100%. The yield of synthesis was 94% for ethyl lactate and 6% for ethyl lactyllactate. 1.26 dm³ of EtOH was distilled-off from reaction mixture at the temperature of 40 °C under a pressure of 45 mbar. 2.2 dm³ of ethyl lactate, yield 92%, was separated by distillation at the temperature of 45 °C under a pressure of 2 mbar.

### 2.6

10.0 g of poly(L-lactide) (100 eq.) in a form of granules from NatureWorks Ingeo™ Biopolymer 2003D, 16.5 ml of anhydrous ethyl alcohol (200 eq.) from Chempur and 0.45 ml 98% Sn(Oct)₂ from Sigma-Aldrich were placed in 150 ml pressure reactor under N2. The reactions were carried out at the temperature of 200 °C for 1h under autogenous pressure of reagents (in a range from atmospheric pressure to 10 bar) with a ratio of [lac_{(PLLA)}]/[Sn]/[EtOH] = 100/1/200. Conversion of PLLA to lactic acid esters was 100%. The yield of synthesis was 90% for ethyl lactate and 10% for ethyl lactyllactate.

### 2.7

10.0 g of poly(L-lactide) (100 eq.) in a form of granules from NatureWorks Ingeo™ Biopolymer 2003D, 16.5 ml of anhydrous ethyl alcohol (200 eq.) from Chempur and 1.39 ml 1.0 M solution of ZnEt₂ in hexane (1 eq.) from Sigma-Aldrich were placed in 150 ml pressure reactor under N₂. The reactions were carried out at the temperature of 200 °C for 1h under autogenous pressure of reagents (in a range from atmospheric pressure to 13 bar) with a ratio of [lac_{(PLLA)}]/[Zn]/[EtOH] = 100/1/200. Conversion of PLLA to lactic acid esters was 100%. The yield of synthesis was 93% for ethyl lactate and 7% for ethyl lactyllactate.

### 2.8

10.0 g of poly(L-lactide) (100 eq.) in a form of granules from NatureWorks Ingeo™ Biopolymer 2003D, 16.5 ml of anhydrous ethyl alcohol (200 eq.) and 0.055g of metallic calcium from Chempur were placed in 150 ml pressure reactor under N₂. The reactions were carried out at the temperature of 200 °C for 1h under autogenous pressure of reagents (in a range from atmospheric pressure to 10 bar) with a ratio of [lac_{(PLLA)}]/[Ca]/[EtOH] = 100/1/200. Conversion of PLLA to lactic acid esters was 100%. The yield of synthesis was 94% for ethyl lactate and 6% for ethyl lactyllactate.

### 2.9

10.0 g of poly(L-lactide) (100 eq.) in a form of granules from NatureWorks Ingeo™ Biopolymer 2003D, 16.5 ml of anhydrous ethyl alcohol (200 eq.) from Chempur and 0.012g of metallic lithium from Sigma-Aldrich were placed in 150 ml pressure reactor under N₂. The reactions were carried out at the temperature of 200 °C for 1h under autogenous pressure of reagents (in a range from atmospheric pressure to 9 bar) with a ratio of [lac_{(PLLA)}]/[Li]/[EtOH] = 100/1/200. Conversion of PLLA to lactic acid esters was 100%. The yield of synthesis was 91% for ethyl lactate and 9% for ethyl lactyllactate.

### 2.10

10.0 g of poly(L-lactide) (100 eq.) in a form of granules from NatureWorks IngeoTM Biopolymer 2003D, 16.5 ml of anhydrous ethyl alcohol (200 eq.) from Chempur and 0.09g of zinc powder from Sigma-Aldrich were placed in 150 ml pressure reactor under N₂. The reactions were carried out at the temperature of 200 °C for 1h under autogenous pressure of reagents (in a range from atmospheric pressure to 20 bar) with a ratio of [lac_{(PLLA)}]/[Zn]/[EtOH] = 100/1/200. Conversion of PLLA to lactic acid esters was 100%. The yield of synthesis was 91% for ethyl lactate and 9% for ethyl lactyllactate.

### 2.11

10.0 g of poly(L-lactide) (100 eq.) in a form of granules from NatureWorks Ingeo™ Biopolymer 2003D, 16.5 ml of anhydrous ethyl alcohol (200 eq.) from Chempur and 0.343g of TiCp₂Cl₂ from Sigma-Aldrich were placed in 150 ml pressure reactor under N₂. The reactions were carried out at the temperature of 200 °C for 1h under autogenous pressure of reagents (in a range from atmospheric pressure to 10 bar) with a ratio of [lac_{(PLLA)}]/[Ti]/[EtOH] = 100/1/200. Conversion of PLLA to lactic acid esters was 100%. The yield of synthesis was 93% for ethyl lactate and 7% for ethyl lactyllactate.

### 2.12

10.0 g of poly(L-lactide) (100 eq.) in a form of granules from NatureWorks Ingeo™ Biopolymer 2003D, 16.5 ml of anhydrous ethyl alcohol (200 eq.) from Chempur and 0.34 g of SnEt₂Cl₂ from Sigma-Aldrich were placed in 150 ml pressure reactor under N₂. The reactions were carried out at the temperature of 200 °C for 1h under autogenous pressure of reagents (in a range from atmospheric pressure to 15 bar) with a ratio of [lac_{(PLLA)}]/[Sn]/[EtOH] = 100/1/200. Conversion of PLLA to lactic acid esters was 100%. The yield of synthesis was 93% for ethyl lactate and 7% for ethyl lactyllactate.

### 2.13

10.0 g of poly(L-lactide) (100 eq.) in a form of granules from NatureWorks Ingeo™ Biopolymer 2003D, 16.5 ml of anhydrous ethyl alcohol (200 eq.) from Chempur and 0.237g of Ti(OMe)₄ from Sigma-Aldrich were placed in 150 ml pressure reactor under N₂. The reactions were carried out at the temperature of 200 °C for 1h under autogenous pressure of reagents (in a range from atmospheric pressure to 9 bar) with a ratio of [lac_{(PLLA)}]/[Ti]/[EtOH] = 100/1/200. Conversion of PLLA to lactic acid esters was 100%. The yield of synthesis was 91% for ethyl lactate and 9% for ethyl lactyllactate.

### 2.14

10.0 g of poly(L-lactide) (100 eq.) in a form of granules from NatureWorks Ingeo™ Biopolymer 2003D, 16.5 ml of anhydrous ethyl alcohol (200 eq.) from Chempur and 0.11g of K(OEt) from Sigma-Aldrich were placed in 150 ml pressure reactor under N₂. The reactions were carried out at the temperature of 200 °C for 1h under autogenous pressure of reagents (in a range from atmospheric pressure to 13 bar) with a ratio of [lac_{(PLLA)}]/[K]/[EtOH] = 100/1/200. Conversion of PLLA to lactic acid esters was 100%. The yield of synthesis was 93% for ethyl lactate and 7% for ethyl lactyllactate.

### 2.15

10.0 g of poly(L-lactide) (100 eq.) in a form of granules from NatureWorks Ingeo™ Biopolymer 2003D, 16.5 ml of anhydrous ethyl alcohol (200 eq.) from Chempur and 0.095 g of Na(OEt) from Sigma-Aldrich were placed in 150 ml pressure reactor under N₂. The reactions were carried out at the temperature of 200 °C for 1h under autogenous pressure of reagents (in a range from atmospheric pressure to 13 bar) with a ratio of [lac_{(PLLA)}]/[Na]/[EtOH] = 100/1/200. Conversion of PLLA to lactic acid esters was 100%. The yield of synthesis was 92% for ethyl lactate and 8% for ethyl lactyllactate.

### 2.16

10.0 g of poly(L-lactide) (100 eq.) in a form of granules from NatureWorks IngeoTM Biopolymer 2003D, 16.5 ml of anhydrous ethyl alcohol (200 eq.) from Chempur and 0.18 g of ZnCl₂ from Sigma-Aldrich were placed in 150 ml pressure reactor under N₂. The reactions were carried out at the temperature of 200 °C for 1h under autogenous pressure of reagents (in a range from atmospheric pressure to 17 bar) with a ratio of [lac_{(PLLA)}]/[Zn]/[EtOH] = 100/1/200. Conversion of PLLA to lactic acid esters was 100%. The yield of synthesis was 91% for ethyl lactate and 9% for ethyl lactyllactate.

### Example 3 The reactions for preparing other esters of lactic and lactylatic acid in the presence of catalyst

### 3.1

30.0 g of poly(L-lactide) (100 eq.) in a form of granules from NatureWorks Ingeo™ Biopolymer 2003D, 62 ml of anhydrous n-propanol (200 eq.) from Sigma-Aldrich and 0.11 g of metallic magnesium (1 eq.) from Chempur were placed in 150 ml pressure reactor under N₂. The reactions were carried out at the temperature of 200 °C for 1.5 h under autogenous pressure of reagents (in a range from atmospheric pressure to 8 bar) with a ratio of [lac_{(PLLA)}]/[Mg]/[nPrOH] = 100/1/200. Conversion of PLLA to lactic acid esters was 100%. The yield of synthesis was 94% for n-propyl lactate and 6% for n-propyl lactyllactate. 30.5 ml of nPrOH was distilled-off from reaction mixture at the temperature of 40 °C under a pressure of 40 mbar. 46 ml of n-propyl lactate, yield 83%, was separated by distillation at the temperature of 45 °C under a pressure of 2 mbar.

### 3.2

30.0 g of poly(L-lactide) (100 eq.) in a form of granules from NatureWorks Ingeo™ Biopolymer 2003D, 76.5 ml of anhydrous sec-butanol (200 eq.) from Sigma-Aldrich and 0.11 g of metallic magnesium (1 eq.) from Chempur were placed in 150 ml pressure reactor under N₂. The reactions were carried out at the temperature of 200 °C for 1.5 h under autogenous pressure of reagents (in a range from atmospheric pressure to 6 bar) with a ratio of [lac_{(PLLA)}]/[Mg]/[secBuOH] = 100/1/200. Conversion of PLLA to lactic acid esters was 100%. The yield of synthesis was 90% for sec-butyl lactate and 10% for sec-butyl lactyllactate. 42 ml of secBuOH was distilled-off from reaction mixture at the temperature of 45 °C under a pressure of 30 mbar. 51 ml of sec-butyl lactate, yield 84%, was separated by distillation at the temperature of 55 °C under a pressure of 2 mbar.

### 3.3

30.0 g of poly(L-lactide) (100 eq.) in a form of granules from NatureWorks Ingeo™ Biopolymer 2003D, 77 ml of anhydrous isobutanol (200 eq.) from Sigma-Aldrich and 0.11 g of metallic magnesium (1 eq.) from Chempur were placed in 150 ml pressure reactor under N₂. The reactions were carried out at the temperature of 200 °C for 2h under autogenous pressure of reagents (in a range from atmospheric pressure to 7 bar) with a ratio of [lac_{(PLLA)}]/[Mg]/[isoBuOH] = 100/1/200. Conversion of PLLA to lactic acid esters was 100%. The yield of synthesis was 95% for isobutyl lactate and 5% for isobutyl lactyllactate. 39.4 ml of isoBuOH was distilled-off from reaction mixture at the temperature of 45 °C under a pressure of 30 mbar. 55.8 ml of isobutyl lactate, yield 89%, was separated by distillation at the temperature of 55 °C under a pressure of 2 mbar.

### 3.4

30.0 g of poly(L-lactide) (100 eq.) in a form of granules from NatureWorks Ingeo™ Biopolymer 2003D, 90.8 ml of anhydrous isopentanol (200 eq.) from Sigma-Aldrich and 0.11 g of metallic magnesium (1 eq.) from Chempur were placed in 150 ml pressure reactor under N₂. The reactions were carried out at the temperature of 200 °C for 2h under autogenous pressure of reagents (in a range from atmospheric pressure to 4 bar) with a ratio of [lac_{(PLLA)}]/[Mg]/[isoPnOH] = 100/1/200. Conversion of PLLA to lactic acid esters was 100%. The yield of synthesis was 90% for isopentyl lactate and 10% for isopentyl lactyllactate. 47.7 ml of isoPnOH was distilled-off from reaction mixture at the temperature of 55 °C under a pressure of 20 mbar. 60.5 ml of isopentyl lactate, yield 87%, was separated by distillation at the temperature of 70 °C under a pressure of 2 mbar.

### 3.5

30.0 g of poly(L-lactide) (100 eq.) in a form of granules from NatureWorks IngeoTM Biopolymer 2003D, 104.6 ml of anhydrous n-hexanol (200 eq.) from Sigma-Aldrich and 0.11 g of metallic magnesium (1 eq.) from Chempur were placed in 150 ml pressure reactor under N2. The reactions were carried out at the temperature of 200 °C for 2h under autogenous pressure of reagents (in a range from atmospheric pressure to 3 bar) with a ratio of [lac_{(PLLA)}]/[Mg]/[nHexOH] = 100/1/200. Conversion of PLLA to lactic acid esters was 100%. The yield of synthesis was 95% for n-hexyl lactate and 5% for n-hexyl lactyllactate. 55 ml of nHexOH was distilled-off from reaction mixture at the temperature of 65 °C under a pressure of 2 mbar. 66 ml of n-hexyl lactate, yield 87%, was separated by distillation at the temperature of 70 °C under a pressure of 2 mbar.

### 3.6

30.0 g of poly(L-lactide) (100 eq.) in a form of granules from NatureWorks Ingeo™ Biopolymer 2003D, 33.7 ml of anhydrous methyl alcohol (200 eq.) and 0.11 g of metallic magnesium (1 eq.) from Chempur were placed in 150 ml pressure reactor under N₂. The reactions were carried out at the temperature of 200 °C for 1.5 h under autogenous pressure of reagents, with a ratio of [lac_{(PLA)}]/[Mg]/[MeOH] = 100/1/200. Conversion of PLA to lactic acid esters was 100%. The yield of synthesis was 94% for methyl lactate and 6% for methyl lactyllactate. 16 ml of MeOH was distilled-off from reaction mixture at the temperature of 30 °C under a pressure of 40 mbar. 32 ml of methyl lactate, yield 85%, was separated by distillation at the temperature of 45 °C under a pressure of 2 mbar.

Elaborated technology will be used in synthesis process of ethyl lactate and other esters of lactic acid that have versatile application in many industrial sectors.

Raw post-reaction mixture, filtrate remaining after the catalyst has been removed or a mixture of few different esters may be used as ready compositions for use in a number of industrial branches. Whereas products of alcoholysis reaction of aliphatic polyesters in the presence of sodium and potassium hydroxide may be used as ready, alkaline surfactant cleaning compositions. The method of the invention falls under the mainstream of research in the field of green chemistry, enabling to prepare a wide range of chemical products without their negative influence on natural environment.

## Claims

1. Method for preparing esters of lactic and lactylatic acid, where the conversion of substrates is 100%, comprising:
a) placement in pressure reactor polylactide (PLA) and alcohol (ROH) wherein the alcohol is selected from methyl, ethyl, n-propyl, isopropyl, n-butyl, isobutyl, sec-butyl, tert-butyl, n-pentyl, isopentyl, n-hexyl, 2-ethylhexyl, n-octyl, allyl, propargyl, benzyl alcohol or mixture of alcohols
with ratio of reagents from 2 to 10 molar equivalents of alcohol for structural unit of polymer ROH/mer;
b) optionally, introducing pre-catalyst to reaction mixture *in situ* wherein the catalyst is prepared in direct one-stage synthesis from organometallic compounds, metal halides, amides, hydrides, carboxylates, acetylacetonates, alcoholates, aryloxanes, metal oxides, metallic dusts, metallic chips, low melt metal alloys and catalytic compositions composed of mixtures of said precursors in the reaction with ROH in solvothermal conditions during the process;
and wherein the catalyst is synthetized from organometallic compounds, hydroxides, metal alkoxides, salts of inorganic acids
c) carrying out the alcoholysis reaction of polylactide in the presence of alcohol or mixture of alcohols, under autogenous pressure of reaction mixture components within a range from atmospheric pressure to 107 bar, in suitably selected temperature, wherein
i. in non-catalyst reaction, the reaction temperature is 220-260°C,
ii. while in catalyst reaction, the reaction temperature is 150-220°C,
d) separating and purifying final product by filtration, evaporation and distillation.

2. The method according to claim 1, **characterized in that** in non-catalyst reaction, the reaction temperature is 240-260°C, and in catalyst reaction, the reaction temperature is 180-220°C.

3. The method according to claim 1, **characterized in that** polylactide (PLA) is selected from the group comprising high molecular weight polylactide, such as isotactic poly(L-lactide) or poly(D-lactide) and atactic poly(D,L-lactide), as well as low molecular weight polylactide.

4. The method according to claim 1, **characterized in that** ester of lactic acid is ethyl lactate and ester of lactylactic acid is ethyl lactylactate.

5. The method according to claim 1, **characterized in that** polylactide is introduced to the reactor in a form of granules or wastes of postconsumer PLA.

6. The method according to claim 1, **characterized in that** alcoholysis of polylactide is conducted under inert atmosphere in solvothermal conditions.

7. The method according to claim 6, **characterized in that** alcoholysis of polylactide is conducted in N₂ or noble gases.

8. The method according to claim 1, **characterized in that** the reaction is conducted in air.

9. The method according to claim 1, **characterized in that** the reaction time is 1 h.

10. The method according to claim 1, **characterized in that** metallic or organometallic pre-catalyst is used in the reaction selected from the group comprising metals of main groups, transition elements and lanthanides, as well as alloys of two and more metals in a composition composed of a mixture of said elements or their organometallic compounds, halides, amides, hydrides, carboxylates, acetylacetonates, metal oxides and their mixture with metallic dust, metallic chips.

11. The method according to claim 10, **characterized in that** organometallic pre-catalyst in a form of solution or in fine crystalline form is used in the reaction.

12. The method according to claim 10, **characterized in that** catalyst is used in the amount of 0.1 to 5% mol per polymer structural unit.

## Patentansprüche

1. Verfahren zur Herstellung von Estern der Milch- und Lactylmilchsäure, wobei die Konversion der Substrate 100% beträgt, umfassend:
a) das Einbringen ein Polylactid (PLA) und einen Alkohol (ROH) in einen Druckreaktor, wobei der Alkohol aus Methyl, Ethyl, n-Propyl, Isopropyl, n-Butyl, Isobutyl, sec-Butyl, tert-Butyl, n-Pentyl, Isopentyl, n-Hexyl, 2-Ethylhexyl, n-Octyl, Allyl, Propargyl, Benzylalkohol oder einer Mischung von Alkoholen ausgewählt ist mit einem Verhältnis der Reagenzien von 2 bis 10 Moläquivalenten des Alkohols zur Struktureinheit des ROH/mer-Polymers;
b) gegebenenfalls das Einführen eines Vorkatalysators in die Reaktionsmischung *in situ,* wobei der Katalysator in einer direkten einstufigen Synthese aus metallorganischen Verbindungen, Metallhalogeniden, Amiden, Hydriden, Carboxylaten, Acetylacetonaten, Alkoholaten, Aryloxanen, Metalloxiden, Metallstäuben, Metallspänen, niedrigschmelzenden Metalllegierungen und den aus Mischungen der genannten Vorläufer bestehenden katalytischen Zusammensetzungen bei der Reaktion mit ROH unter Solvothermalbedingungen während des Prozesses hergestellt wird;
und wobei der Katalysator aus metallorganischen Verbindungen, Hydroxiden, Metallalkoxiden, Salzen anorganischer Säuren synthetisiert ist
c) das Durchführen der Alkoholysereaktion vom Polylactid in Gegenwart eines Alkohols oder einer Mischung von Alkoholen unter autogenem Druck der Reaktionsmischungskomponenten im Bereich von Atmosphärendruck bis 107 bar, bei entsprechend gewählter Temperatur, wobei
i. bei der Nicht-Katalysator-Reaktion die Reaktionstemperatur 220-260°C beträgt,
ii. während bei der Katalysator-Reaktion die Reaktionstemperatur 150-220°C beträgt,
d) das Abtrennen und Reinigen des Endproduktes durch Filtration, Verdampfung und Destillation.

2. Verfahren nach Anspruch 1, **dadurch gekennzeichnet, dass** bei der Nicht-Katalysator-Reaktion die Reaktionstemperatur 240-260°C beträgt und bei der Katalysator-Reaktion die Reaktionstemperatur 180-220°C beträgt.

3. Verfahren nach Anspruch 1, **dadurch gekennzeichnet, dass** das Polylactid (PLA) aus der Gruppe umfassend ein hochmolekulares Polylactid, wie isotaktisches Poly-L-Lactid oder Poly-D-Lactid und ataktisches Poly-D,L-Lactid, sowie ein niedermolekulares Polylactid ausgewählt ist.

4. Verfahren nach Anspruch 1, **dadurch gekennzeichnet, dass** der Ester der Milchsäure ein Ethyllactat ist und der Ester der Lactylmilchsäure ein Ethyllactyllactat ist.

5. Verfahren nach Anspruch 1, **dadurch gekennzeichnet, dass** das Polylactid in Form von Granulat oder Abfällen von Post-Consumer-PLA in den Reaktor eingeführt wird.

6. Verfahren nach Anspruch 1, **dadurch gekennzeichnet, dass** die Alkoholyse des Polylactids unter einer inerten Atmosphäre unter Solvothermalbedingungen durchgeführt wird.

7. Verfahren nach Anspruch 6, **dadurch gekennzeichnet, dass** die Alkoholyse des Polylactids in N₂ oder Edelgasen durchgeführt wird.

8. Verfahren nach Anspruch 1, **dadurch gekennzeichnet, dass** die Reaktion an der Luft durchgeführt wird.

9. Verfahren nach Anspruch 1, **dadurch gekennzeichnet, dass** die Reaktionszeit 1 h beträgt.

10. Verfahren nach Anspruch 1, **dadurch gekennzeichnet, dass** ein metallischer oder metallorganischer Vorkatalysator, ausgewählt aus der Gruppe umfassend Metalle der Hauptgruppen, Übergangselemente und Lanthanoide sowie Legierungen aus zwei und mehr Metallen in einer Zusammensetzung bestehend aus einer Mischung der genannten Elemente oder deren metallorganischen Verbindungen, Halogeniden, Amiden, Hydriden, Carboxylaten, Acetylacetonaten, Metalloxiden und deren Mischung mit Metallstaub, Metallspänen, bei der Reaktion verwendet wird.

11. Verfahren nach Anspruch 10, **dadurch gekennzeichnet, dass** der metallorganische Vorkatalysator in Form einer Lösung oder in feinkristalliner Form bei der Reaktion verwendet wird.

12. Verfahren nach Anspruch 10, **dadurch gekennzeichnet, dass** der Katalysator in einer Menge von 0,1 bis 5 Mol-% pro Polymerstruktureinheit verwendet wird.

## Revendications

1. Procédé de préparation d'esters d'acide lactique et lactylatique, dans lequel la conversion de substrats est de 100%, comprenant:
a) mise en place dans un réacteur sous pression de polylactide (PLA) et d'alcool (ROH), l'alcool étant choisi parmi alcool méthylique, éthylique, n-propylique, isopropylique, n-butylique, isobutylique, sec-butylique, tert-butylique, n-pentylique, isopentylique, n-hexylique, 2-éthylhexylique, n-octylique, allylique, propargylique, benzylique ou un mélange d'alcools
avec un rapport de réactifs de 2 à 10 équivalents molaires d'alcool pour l'unité structurelle du polymère ROH/mer;
b) éventuellement, introduction d'un pré-catalyseur dans un mélange réactionnel *in situ,* le catalyseur étant préparé en une seule étape de synthèse directe à partir de composés organométalliques, d'halogénures métalliques, d'amides, d'hydrures, de carboxylates, d'acétylacétonates, d'alcoolates, d'aryloxanes, d'oxydes métalliques, de poussières métalliques, de copeaux métalliques, d'alliages métalliques à faible point de fusion et de compositions catalytiques composées de mélanges desdits précurseurs dans la réaction avec ROH dans des conditions solvothermiques au cours du procédé;
et dans lequel le catalyseur est synthétisé à partir de composés organométalliques, d'hydroxydes, d'alcoxydes métalliques, de sels d'acides inorganiques
c) réalisation de la réaction d'alcoolyse du polylactide en présence d'un alcool ou d'un mélange d'alcools, sous pression autogène des composants du mélange réactionnel dans une plage allant de la pression atmosphérique à 107 bars, à une température convenablement choisie, dans lequel
i. dans une réaction non catalytique, la température de réaction est de 220-260°C,
ii. tandis que dans la réaction catalytique, la température de la réaction est de 150-220°C,
d) séparation et purification du produit final par filtration, évaporation et distillation.

2. Procédé selon la revendication 1, **caractérisé en ce que**, dans une réaction non catalytique, la température de réaction est de 240 à 260°C, et dans une réaction catalytique, la température de réaction est de 180 à 220°C.

3. Procédé selon la revendication 1, **caractérisé en ce que** le polylactide (PLA) est choisi dans le groupe comprenant le polylactide de haut poids moléculaire, tel que le poly(L-lactide) isotactique ou le poly(D-lactide) et le poly(D,L-lactide) atactique, ainsi que le polylactide de bas poids moléculaire.

4. Procédé selon la revendication 1, **caractérisé en ce que** l'ester de l'acide lactique est le lactate d'éthyle et l'ester de l'acide lactylactique est le lactylactate d'éthyle.

5. Procédé selon la revendication 1, **caractérisé en ce que** le polylactide est introduit dans le réacteur sous forme de granulés ou de déchets de PLA post-consommation.

6. Procédé selon la revendication 1, **caractérisé en ce que** l'alcoolyse du polylactide est effectuée sous atmosphère inerte dans des conditions solvothermiques.

7. Procédé selon la revendication 6, **caractérisé en ce que** l'alcoolyse du polylactide est effectuée dans du N₂ ou des gaz nobles.

8. Procédé selon la revendication 1, **caractérisé en ce que** la réaction est effectuée dans l'air.

9. Procédé selon la revendication 1, **caractérisé en ce que** le temps de réaction est de 1 h.

10. Procédé selon la revendication 1, **caractérisé en ce que** le pré-catalyseur métallique ou organométallique utilisé dans la réaction est choisi dans le groupe comprenant les métaux des groupes principaux, les éléments de transition et les lanthanides, ainsi que les alliages de deux métaux et plus dans une composition composée d'un mélange desdits éléments ou de leurs composés organométalliques, les halogénures, les amides, les hydrures, les carboxylates, les acétylacétonates, les oxydes métalliques et leur mélange avec les poussières métalliques, les copeaux métalliques.

11. Procédé selon la revendication 10, **caractérisé en ce que** le pré-catalyseur organométallique sous forme de solution ou sous forme cristalline fine est utilisé dans la réaction.

12. Procédé selon la revendication 10, **caractérisé en ce que** le catalyseur est utilisé en une quantité de 0,1 à 5% en moles par unité structurelle polymère.
